# FASCICULE DE BREVET EUROPEEN

(11) **EP 4 205 800 B1**
(45) Date de publication et mention de la délivrance du brevet: **03.04.2024**
(21) Numéro de dépôt: 22153565.1
(22) Date de dépôt: 27.01.2022
(51) Int. Cl.: A61N 1/05, A61N 1/375

(54) **DISPOSITIF MÉDICAL IMPLANTABLE À VIS D'ANCRAGE HÉLICOÏDALE FLEXIBLE**
IMPLANTIERBARE MEDIZINISCHE VORRICHTUNG MIT FLEXIBLER HELIXFÖRMIGER VERANKERUNGSSCHRAUBE
IMPLANTABLE MEDICAL DEVICE WITH FLEXIBLE HELICAL ANCHORING SCREW

(30) Priorité: 29.12.2021 EP 21315298
(43) Date de publication de la demande: 05.07.2023
(73) Titulaire: CAIRDAC, 92160 Antony (FR)
(72) Inventeur: REGNIER, Willy, 91160 Longjumeau (FR); NGUYEN-DINH, An, 37520 La Riche (FR)
(74) Mandataire: Dupuis-Latour, Dominique

(56) Documents cités:
- EP-B1- 2 473 228
- US-A- 4 311 153
- US-A1- 2011 307 043
- US-A1- 2019 076 664
- US-A1- 2020 197 705
- US-A1- 2020 289 835
- US-B2- 11 197 997

## Description

### Domaine de l'invention

L'invention concerne les dispositifs médicaux implantés, en particulier ceux de ces dispositifs qui sont destinés à être implantés dans une cavité cardiaque.

Comme on l'exposera par la suite, l'invention est de façon particulièrement avantageuse applicable à des dispositifs implantables autonomes de type "capsule *leadless*", qui sont des dispositifs implantables dépourvus de toute liaison physique (*lead*) à un dispositif distant.

L'invention n'est toutefois pas limitée à un type particulier de capsule, ni même d'implant, et elle est applicable indifféremment à de nombreux autres types de dispositifs, quelle que soit leur destination fonctionnelle, cardiaque ou autre, par exemple à des capsules destinées à diffuse *in situ* un agent pharmacologique actif.

Dans le cas d'une application cardiaque, la capsule surveille en continu le rythme du patient et délivre si nécessaire au coeur des impulsions électriques de stimulation, de resynchronisation et/ou de défibrillation en cas de trouble du rythme détecté par la capsule. La capsule comprend divers circuits électroniques, capteurs, etc., ainsi que des moyens émetteurs/récepteurs de communication sans fil pour l'échange de données à distance, le tout étant intégré dans un corps de très petites dimensions qui puisse être implanté dans des sites difficilement accessibles ou laissant peu d'espace, tels que l'apex du ventricule ou la paroi interne de l'oreillette.

En particulier, la mise en place d'une capsule auriculaire présente le très grand avantage de pouvoir mettre en oeuvre une stimulation de type "double chambre" combinant une capsule ventriculaire et une capsule auriculaire munies de moyens de communication mutuels. La capsule auriculaire détecte les dépolarisations auriculaires du rythme sinusal, et la capsule ventriculaire, ainsi qu'éventuellement la capsule auriculaire, délivre(nt) en tant que de besoin au ventricule et/ou à l'oreillette des impulsions de stimulation séquencées de manière à contrôler de façon précise le délai atrioventriculaire de stimulation.

L'invention concerne plus particulièrement l'ancrage de la capsule, ou plus généralement du dispositif médical, au site d'implantation choisi.

La capsule est munie à son extrémité distale d'un organe d'ancrage apte à pénétrer dans les tissus d'une paroi corporelle. Dans le cas d'une capsule endocavitaire, la capsule est fixée à la paroi intérieure d'une cavité ventriculaire ou auriculaire.

Il existe plusieurs types d'ancrage, souvent à base de barbes (*tines*) élastiquement déformables se déployant dans la masse du myocarde, mais aussi des ancrages par une vis hélicoïdale saillante prolongeant axialement le corps de la capsule et conçue pour pénétrer dans le tissu cardiaque au site d'implantation par vissage. L'invention concerne ce dernier type d'organe d'ancrage, à vis hélicoïdale.

La "délivrance" de la capsule, c'est-à-dire sa mise en place au site d'implantation, consiste à monter la capsule à l'extrémité d'un cathéter-guide d'un accessoire d'implantation, puis à la faire progresser le long du réseau veineux périphérique et la diriger ensuite jusqu'au site choisi, par exemple l'apex de la cavité ventriculaire droite. Une fois le site d'implantation atteint, par l'intermédiaire du cathéter-guide le praticien imprime à la capsule un mouvement combiné de translation axiale (pour faire progresser la capsule vers l'avant puis exercer une pression contre la paroi cardiaque) et de rotation de la capsule sur elle-même (pour visser l'organe d'ancrage dans l'épaisseur de la paroi cardiaque). Une fois la capsule fermement ancrée, le praticien procède au "largage" de la capsule, c'est-à-dire à sa désolidarisation d'avec l'accessoire d'implantation, la capsule devenant alors totalement autonome.

### Description de la technique antérieure

Le WO 2002/064172 A1 (Cairdac) (ou US 11 197 997 B2 (Regnier)), décrit une capsule munie d'une vis d'ancrage hélicoïdale, et pourvue en outre d'un système de limitation de couple permettant de débrayer le corps de la capsule de la vis d'ancrage lorsque le couple de réaction exercé par la vis d'ancrage dépasse un seuil prédéterminé, de manière à ne pas dépasser une valeur limite dite "coupe de carottage" au-delà de laquelle la vis d'ancrage risquerait de déchirer localement les tissus sous l'effet de la rotation de la vis sans avance de celle-ci, jusqu'à provoquer une lacération des tissus et, à l'extrême, une perforation de la paroi avec risque de tamponnade.

Le EP 2 473 228 B1 (Mayo Foundation) décrit un câble électrique pour un dispositif cardiaque implantable, le câble comprenant une pointe spécialement aménagée.

Le US 2011/307043 A1 (Sorin CRM) décrit un système comprenant une capsule intracardiaque autonome ainsi que son dispositif d'implantation.

Comme on l'expliquera en détail par la suite, ce risque d'endommagement des tissus est particulièrement élevé dans le cas d'une implantation dans une paroi mince telle que celle de l'oreillette, notamment dans la région de l'appendice auriculaire droit ou RAA (*Right Atrial Appendage*), compte tenu de l'épaisseur très faible, typiquement de l'ordre de 0,5 à 2 mm suivant l'emplacement. La difficulté est accrue par le fait que la zone du RAA contient de nombreux replis et qu'il n'est pas possible de prédire l'épaisseur exacte au site d'implantation avant le vissage et que de plus, en cas de perforation de la paroi cardiaque, il n'est pas possible de déceler un saignement, l'épanchement du sang se produisant à l'intérieur du sac péricardique (tamponnage hémorragique). De plus, du fait de ces replis, il n'est pas possible de déterminer exactement l'angle que formera la capsule avec le myocarde au moment du contact avec la paroi cardiaque. Si la direction est oblique, le risque de perforation est moindre mais en revanche le risque de mauvais seuil de détection sera accru, et inversement.

En tout état de cause, compte tenu de l'épaisseur réduite de la paroi, la longueur fixante de l'hélice (longueur de la vis au-delà de la face plane frontale de la capsule supportant l'électrode de détection/stimulation) devra nécessairement être inférieure à 1,5 mm, qui est une longueur réduite qui rend difficile l'obtention d'un ancrage fiable et durable du dispositif à la paroi de l'oreillette.

Le but de la présente invention est de proposer un dispositif médical implantable muni d'un ancrage à vis permettant une fixation mécaniquement sûre et cliniquement sans risque à une paroi mince, en faisant en sorte de ne pas traverser la paroi mince de l'organe avec l'hélice - notamment, dans le cas d'une implantation dans l'oreillette, de préserver l'espace péricardique et prévenir tout saignement.

### RÉSUMÉ DE L'INVENTION

Pour résoudre ces problèmes et atteindre les buts exposés ci-dessus, l'invention propose à cet effet un dispositif médical implantable selon le WO 2002/064172 A1 précité, comprenant un corps de dispositif avec une face frontale à son extrémité distale, et un moyen d'ancrage du dispositif médical à une paroi d'un organe d'un patient. Le moyen d'ancrage comporte une vis avec un fil d'hélice enroulé en une pluralité de spires non jointives, la vis ayant une extrémité encastrée solidarisée à la face frontale du corps de dispositif et une extrémité libre comprenant une extrémité biseautée définie par au moins une surface oblique.

De façon caractéristique de l'invention, le fil d'hélice comprend à l'extrémité distale libre du fil d'hélice une région terminale dont la raideur en flexion du fil d'hélice est plus faible que dans une région proximale des spires du fil d'hélice.

Selon diverses caractéristiques subsidiaires avantageuses :
- la région terminale s'étend sur au moins une demi-spire de l'hélice ;
- la région terminale est une région de raideur non constante comprenant une pluralité de portions successives différentes dont les raideurs respectives vont en décroissant dans un sens proximal vers distal ;
- la région terminale est une région de raideur non constante comprenant un fil d'hélice de diamètre variable, continûment décroissant dans un sens proximal vers distal ;
- dans ce dernier cas, le diamètre du fil d'hélice peut être en particulier un diamètre variable sur une longueur d'une spire du fil d'hélice, puis constant sur les spires proximales suivantes de l'hélice, notamment un diamètre qui augmente de 0,2 mm côté distal de l'extrémité terminale, jusqu'à 0,5 mm côté proximal de l'extrémité terminale ;
- la région terminale est une région de raideur non constante, comprenant une pluralité de portions successives différentes de même diamètre ayant subi des traitements thermiques propres à modifier leurs raideurs respectives ;
- le fil d'hélice de la vis présente un pas d'enroulement à vide constant, ou bien, au moins sur la longueur de la région terminale, un pas d'enroulement à vide variable, décroissant dans un sens distal vers proximal ;
- le dispositif comprend en outre un couplage débrayable agencé pour, lorsqu'une sollicitation externe en rotation axiale est appliquée au corps de dispositif dans un sens de vissage de la vis, autoriser une rotation relative axiale entre le corps de dispositif et le moyen d'ancrage dès qu'un couple de réaction exercé par la vis dans la paroi de l'organe du patient dépasse un couple seuil prédéterminé.

Le dispositif de l'invention peut notamment être un implant cardiaque autonome de type capsule *leadless,* dans lequel le corps de dispositif loge un ensemble électronique et un module de récupération d'énergie avec un moyen de stockage d'énergie pour l'alimentation de l'ensemble électronique, et dans lequel la face frontale du corps de dispositif porte une électrode de détection/stimulation cardiaque apte à venir en contact avec la paroi de l'organe du patient après ancrage du dispositif.

### DESCRIPTION SOMMAIRE DES DESSINS

On va maintenant décrire un exemple de réalisation de la présente invention en référence aux dessins annexés, où les mêmes références désignent d'une figure à l'autre des éléments identiques ou fonctionnellement semblables.
La Figure 1 illustre des dispositifs médicaux de type capsule *leadless* dans leur environnement, avec divers exemples de sites d'implantation dans, sur ou à proximité du coeur d'un patient.
La Figure 2 est une vue d'ensemble extérieure d'une capsule *leadless,* montrant notamment la vis d'ancrage et l'électrode de stimulation à son extrémité distale.
La Figure 3 montre plus précisément l'extrémité distale d'une capsule *leadless* munie d'une vis d'ancrage selon l'état de la technique, avec l'extrémité de la vis à l'approche de la paroi du site d'implantation.
La Figure 4 est une vue de détail de l'extrémité biseautée de la vis d'ancrage de la Figure 3.
La Figure 5 est une vue en coupe d'une paroi cardiaque, notamment au niveau de l'oreillette, détaillant les différents tissus qui constituent cette paroi.
La Figure 6 est une vue en perspective d'une vis d'ancrage selon la présente invention.
La Figure 7 est un détail de l'extrémité distale de la vis d'ancrage de la Figure 6.
La Figure 8 illustre une variante de la Figure 7.
La Figure 9 illustre un mode de réalisation selon les enseignements de l'invention, dans lequel la vis d'ancrage présente une raideur en flexion qui n'est pas constante dans une région terminale située au voisinage de l'extrémité distale de l'hélice.
Les Figures 10 et 11 illustrent deux lois de variation possibles de la raideur en flexion sur une fraction correspondante de la région terminale.
La Figure 12 illustre schématiquement les différentes parties du fil d'hélice à usiner pour obtenir une vis d'ancrage hélicoïdale à raideur en flexion variable selon l'invention.
La Figure 13 est un schéma d'étapes de processus pour l'obtention d'une vis d'ancrage hélicoïdale selon l'invention.
La Figure 14 illustre un exemple d'outil permettant de réaliser l'étape d'amincissement du fil du processus de la Figure 13.
La Figure 15 montre l'extrémité distale d'une vis d'ancrage selon l'invention, pendant la pénétration de la paroi cardiaque au site d'implantation.
La Figure 16 est une vue schématique agrandie de l'extrémité biseautée de la vis d'ancrage selon l'invention, explicitant les différentes forces exercées au niveau de cette extrémité, ainsi que leur résultante.

### DESCRIPTION DÉTAILLÉE DE MODES DE RÉALISATION PRÉFÉRENTIELS DE L'INVENTION

On va maintenant décrire un exemple de réalisation du dispositif de l'invention, dans une application à une capsule implantable autonome destinée à être implantée dans une cavité cardiaque.

Comme on l'a indiqué plus haut, cette application particulière n'est donnée qu'à titre d'exemple de réalisation et n'est pas limitative de l'invention, dont les enseignements peuvent être appliqués à de nombreux autre types de dispositifs implantables.

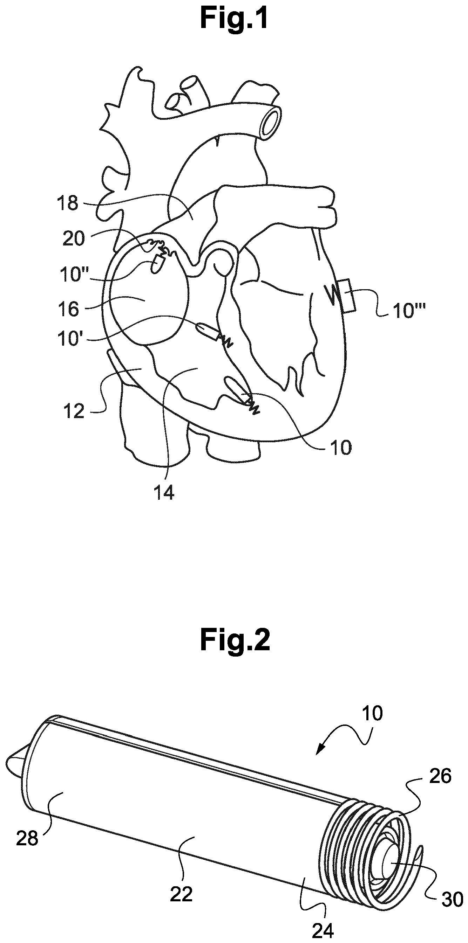

Sur la Figure 1, on a représenté diverses possibilités de sites d'implantation d'un dispositif médical implantable de type capsule *leadless,* dans une application à la stimulation cardiaque. Ainsi, la capsule référencée 10 est implantée à l'apex du ventricule droit 14 du myocarde 12. La capsule peut être également implantée sur le septum interventriculaire, comme en 10', ou encore sur une paroi de l'oreillette droite 16, comme en 10". Le dispositif peut également être une capsule épicardique placée sur une région externe du myocarde, comme illustré en 10'".

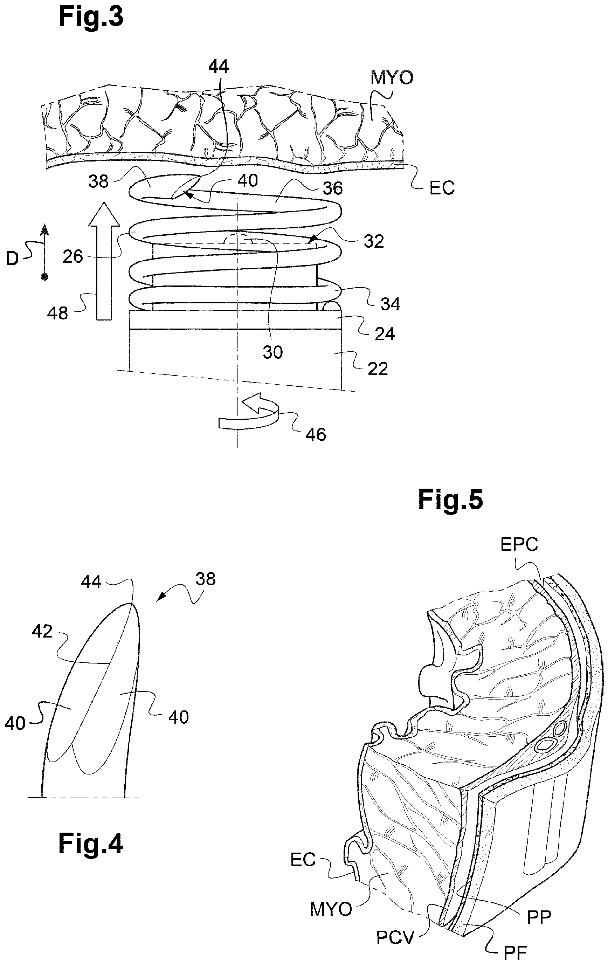

On décrira plus en détail par la suite, en référence notamment à la Figure 5, le cas spécifique d'une implantation dans la région de l'appendice auriculaire droit 18, où la paroi cardiaque à l'intérieur de l'oreillette 16 présente une épaisseur plus réduite, ainsi qu'un certain nombre de replis et trabéculations 20 ; cette région présente l'avantage de ne pas se situer dans le prolongement de la veine cave supérieure et de la valve tricuspide, de sorte que la présence d'une capsule à cette endroit ne gênera pas l'accès aux autres zones des cavités auriculaire ou ventriculaire, en particulier pour accéder au fond du ventricule si l'on veut implanter ou explanter une capsule dans la région de l'apex.

Dans tous les cas, la capsule *leadless* est fixée à la paroi cardiaque au moyen d'un système d'ancrage saillant pénétrant dans le tissu cardiaque pour la maintenir en place au site d'implantation. Divers systèmes sont utilisables, l'invention concernant plus particulièrement les capsules munies d'un organe d'ancrage à vis hélicoïdale.

Comme illustré sur la Figure 2, une capsule munie d'un tel organe se présente globalement sous la forme d'un corps tubulaire 22 enfermant les divers circuits électroniques et d'alimentation de la capsule. Les dimensions typiques des capsules connues sont un diamètre de l'ordre de 6 mm pour une longueur d'environ 25 à 40 mm. Le corps tubulaire 22 comporte à son extrémité avant (distale) 24 une vis hélicoïdale cylindrique 26 destinée à maintenir la capsule en place au site d'implantation. L'extrémité arrière opposée (proximale) 28 est une extrémité libre, qui est seulement pourvue de moyens (non représentés) de liaison temporaire à un cathéter-guide ou autre accessoire d'implantation utilisable pour la mise en place ou l'explantation de la capsule et qui est ensuite désolidarisé de cette dernière.

Pour assurer les fonctions de détection/stimulation, une électrode 30 est disposée sur la face frontale de la capsule ; une fois la capsule ancrée au site d'implantation, l'électrode 30 est en contact avec le tissu cardiaque, permettant ainsi le recueil des potentiels de dépolarisation et/ou l'application d'impulsions de stimulation.

La Figure 3 montre plus précisément l'extrémité distale de la capsule avec la vis hélicoïdale 26 à l'approche de la paroi cardiaque, comprenant le myocarde MYO et l'endocarde EC.

Au moment de l'implantation, la face frontale 32 du corps 22 de la capsule, qui porte l'électrode 30, est tournée vers la paroi de l'endocarde. La vis hélicoïdale 26 comprend, côté proximal, des spires inactives 34 solidaires du corps 22 de la capsule et, à l'opposé, des spires actives 36 terminées par une extrémité libre biseautée 38.

Une telle capsule est par exemple décrite par le WO 2002/064172 A1 (US 11 197 997 B2) précité.

La Figure 4 illustre plus précisément la pointe biseautée 38, qui est une pointe perforante comprenant une ou plusieurs surfaces obliques 40 permettant de créer une arête coupante 42 avec une pointe perforante 44 à l'extrémité la plus distale de la vis hélicoïdale. Comme on peut le voir sur la Figure 3, l'extrémité perforante 44 est dirigée vers la paroi cardiaque, avec les surfaces obliques 40 tournées vers la direction proximale (la direction distale étant indiquée en D sur la Figure 3).

Cette configuration avec la pointe tournée vers la paroi (c'est-à-dire orientée en direction distale D) facilite la perforation de l'endocarde EC puis la pénétration dans le myocarde MYO dans les différentes configurations susceptibles d'être rencontrées, même en cas d'inclinaison entre l'axe de la capsule et la normale à la paroi au moment de la venue en contact avec cette dernière. Une fois la pointe 44 en contact avec l'endocarde EC, le mouvement combiné de rotation 46 et de translation 48 (poussée axiale) exercé par le praticien via le cathéter-guide assure la perforation de l'endocarde EC et la pénétration de la vis dans le myocarde MYO sur la longueur des spires actives 36, qui est de l'ordre de 1,5 mm ou plus pour une fixation dans la cavité ventriculaire. La face frontale 32 portant l'électrode 30 est alors en contact avec la paroi cardiaque, permettant à l'électrode d'assurer ses fonctions de détection/stimulation.

La configuration connue de vis d'ancrage que l'on vient de décrire en référence aux Figures 3 et 4 est toutefois mal adaptée à une fixation dans l'oreillette, car il faut prendre garde à ne pas perforer la paroi cardiaque. La Figure 5 illustre les différents tissus constituant la paroi cardiaque de l'oreillette, notamment au niveau de l'appendice auriculaire droit (RAA) : la paroi comprend, de l'intérieur vers l'extérieur, l'endocarde EC, le myocarde MYO, le péricarde viscéral PCV, l'espace péricardique EPC, le péricarde pariétal PP et le péricarde fibreux PF.

L'épaisseur totale de la paroi est comprise entre 0,5 et 2 mm, donc une valeur beaucoup plus faible que celle du ventricule, que ce soit au niveau de l'apex ou du septum. De plus, le péricarde viscéral PCV est une paroi très fine, d'épaisseur environ 0,2 mm, qui est traversée par endroits par des vaisseaux sanguins coronaires. Si l'on veut implanter dans une telle paroi une capsule munie d'un organe d'ancrage à vis, il ne faut surtout pas traverser le péricarde viscéral PCV avec l'hélice de la vis, afin d'éviter toute perforation qui créerait un risque de tamponnade hémorragique avec épanchement de sang dans l'espace péricardique EPC. Cela étant, il est nécessaire de pénétrer suffisamment le myocarde MYO pour garantir une bonne fixation de la capsule à la paroi cardiaque.

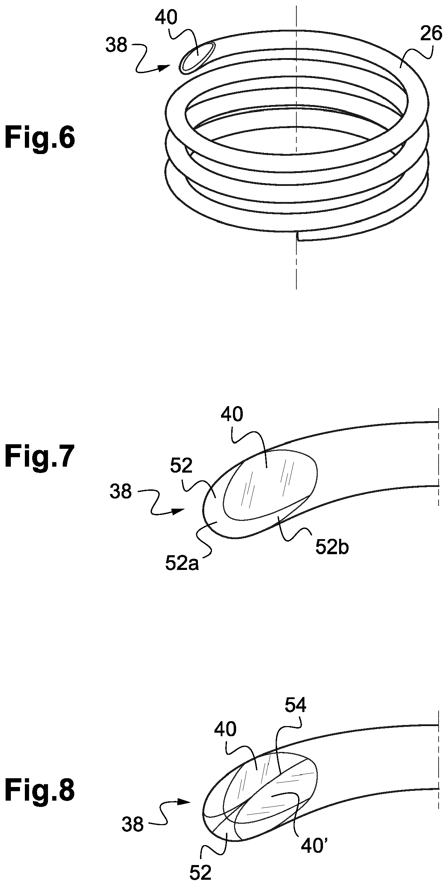

Pour résoudre ce problème, l'invention propose une nouvelle configuration de vis d'ancrage, illustrée aux Figures 6 à 16.

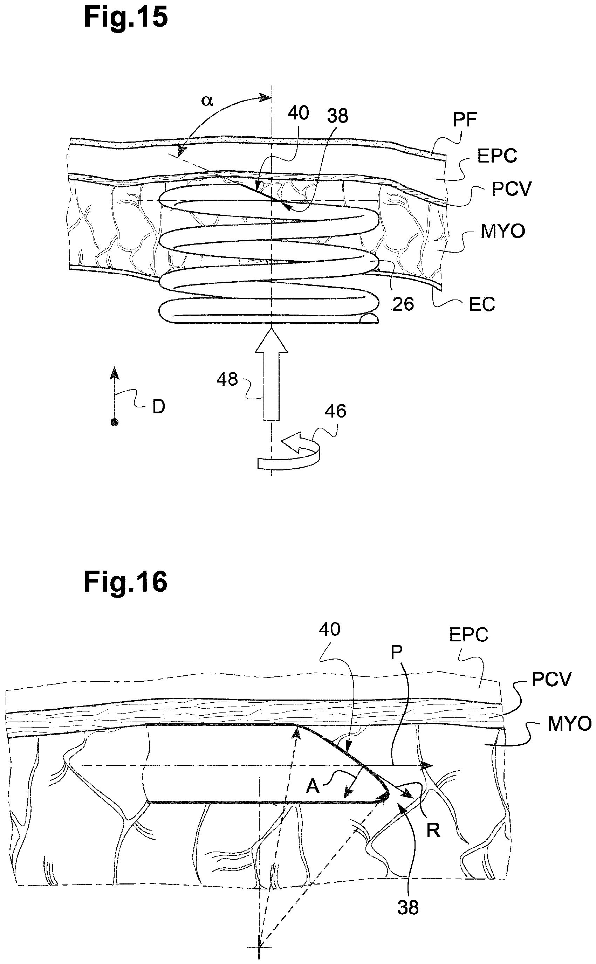

Comme illustré Figure 6, la vis hélicoïdale 26 est terminée par une extrémité libre biseautée 38 définie par au moins une surface oblique 40. La surface oblique 40 peut être une surface plane. Elle peut également être une surface courbe, par exemple une surface conique telle que la surface d'un cône de révolution ou d'un cône oblique dont la région voisine du sommet aurait été émoussée pour ne pas être traumatique. Très préférentiellement, la surface oblique 40 définissant le biseau de l'extrémité 38 est tournée en direction de la paroi, c'est-à-dire en direction distale D (cf. Figure 15). En d'autres termes, la normale à la surface oblique 40 est orientée dans une direction opposée à la face frontale du corps du dispositif, de manière que cette surface oblique 40 soit tournée vers la paroi de l'organe - à la différence des vis d'ancrage de l'état de la technique, où la surface oblique du biseau est tournée dans une direction opposée à la paroi (comme illustré Figure 3).

La normale à la surface oblique forme un angle α (cf. Figure 15) compris typiquement entre 30° et 60° par rapport à l'axe de l'hélice.

Une autre caractéristique préférentielle de la vis hélicoïdale d'ancrage selon l'invention est qu'elle est élastiquement déformable en compression axiale, avec un coefficient de raideur d'au plus 5 N/mm.

Le fil d'hélice peut notamment présenter les caractéristiques suivantes (qui sont en elles-mêmes non limitatives de l'invention) :
- enroulement à droite ;
- matériau biocompatible tel qu'acier inoxydable, titane, nitinol, etc., avantageusement avec un revêtement procurant un état de surface formant des microreliefs (revêtement de nitrure de titane) ou une porosité (par attaque chimique) qui favorisent après implantation l'adhésion de la vis à des tissus fibrotiques ;
- diamètre du fil : 0,2 à 1 mm ;
- diamètre extérieur de l'hélice : inférieur à 26 French (8,67 mm) ;
- nombre de spires actives : 1 à 5 spires ;
- pas d'enroulement à vide : 1 mm (le pas pouvant éventuellement être un pas variable) ;
- longueur des spires actives en direction axiale : inférieure à 1,5 mm.

L'extrémité biseautée 38 est réalisée par un usinage permettant de façonner la surface oblique 40. Avantageusement, comme illustré Figure 7, un arrondi 52 est confectionné sur le pourtour de la surface oblique 40, par usinage traditionnel ou par des tirs laser. Le bord arrondi 52 permet de supprimer l'arête coupante (que l'on avait avec les vis hélicoïdales de l'art antérieur telles que celles illustrées Figures 3 et 4) et de rendre atraumatique la géométrie de l'extrémité de la vis hélicoïdale. On prévient ainsi la perforation du péricarde viscéral en supprimant toute pointe ou arête coupante à l'extrémité de l'hélice. Avantageusement, l'arrondi formé à l'extrémité de l'hélice est un arrondi variable, avec une valeur de rayon diminuant progressivement depuis l'extrémité distale 52a jusqu'à la région la moins distale 52b. Le diamètre de l'arrondi à l'extrémité distale 52a est par exemple de 0,1 mm, de façon générale un diamètre d'arrondi inférieur à 1/5 du diamètre du fil.

La Figure 8 illustre une variante de la Figure 7, dans laquelle l'extrémité biseautée 38 comprend deux surfaces obliques 40, 40' formant à leur intersection une arête 54, également arrondie de manière à n'être pas coupante.

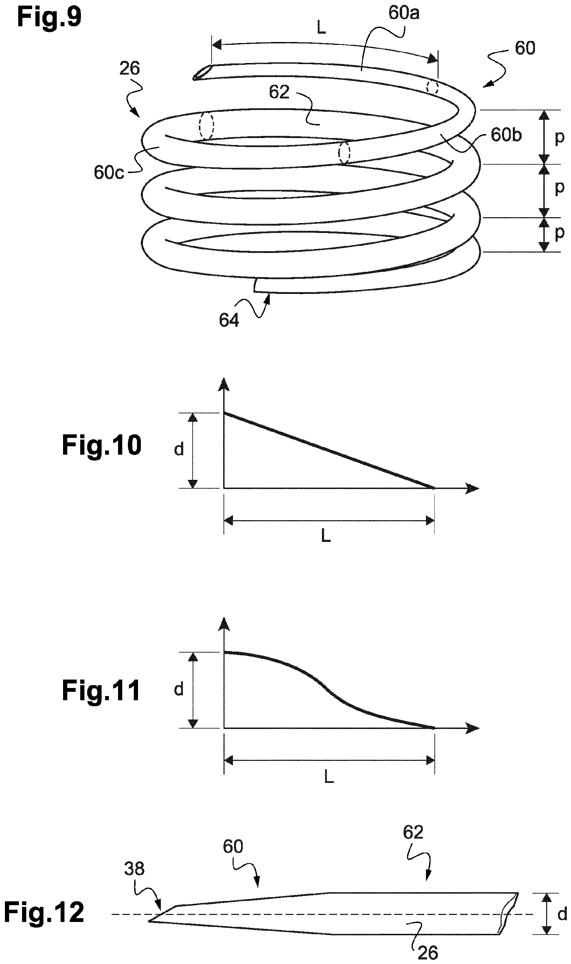

La Figure 9 illustre un mode de réalisation selon les enseignements de l'invention, dans lequel, dans une région terminale 60 située au voisinage de l'extrémité distale 38 de l'hélice, la vis d'ancrage 26 présente une raideur en flexion qui n'est pas la même que dans la partie 62 la plus proximale de l'hélice.

À cet effet, le fil est constitué d'une ou plusieurs zones ayant des raideurs en flexion différentes, par exemple, comme dans l'exemple illustré, une juxtaposition de trois portions successives 60a, 60b, 60c de raideurs respectives différentes, croissantes dans le sens distal vers proximal ; c'est-à-dire que le fil est plus déformable en flexion (sa raideur est moindre) côté distal que côté proximal.

Plusieurs modes de réalisation peuvent être envisagés pour obtenir ces raideurs en flexion différentes dans la région terminale.

Dans la variante (non limitative) illustrée Figure 9, la partie terminale 60 à moindre raideur en flexion est constituée d'une pluralité de portions de fil 60a, 60b, 60c dont chacune a un diamètre qui décroît de son extrémité distale à son extrémité proximale. Toujours dans cette variante, la partie terminale 60 comprend trois portions de fil 60a, 60b, 60c de même longueur L, s'étendant sur un tiers de spire chacune. La portion la plus distale 60a a un diamètre de fil qui croît par exemple de 0,2 à 0,3 mm dans le sens distal vers proximal, la portion intermédiaire 60b un diamètre qui croît de 0,3 à 0,4 mm, et la portion la plus proximale 60c un diamètre qui croît de 0,4 à 0,5 mm. Au-delà, dans la partie 62 la plus proximale de l'hélice 26, le diamètre du fil est un diamètre constant de 0,5 mm.

Les diamètres successifs croissants donnent au fil une raideur correspondante croissante, par exemple une raideur K qui varie de 0,1 N/mm (à l'extrémité la plus distale de l'hélice) à 0,6 N/mm (dans la partie la plus proximale, là où le diamètre du fil d'hélice est maximal puis constant).

Les indications et valeurs numériques données ci-dessus ne le sont bien entendu qu'à titre d'illustration, sans aucun caractère limitatif.

Par exemple, la partie terminale 60 à raideur variable peut s'étendre sur plus, ou moins, que la longueur d'une spire comme dans le cas de la Figure 9.

De même, le nombre des portions successives peut être différent, par exemple quatre sections d'un quart de spire chacune au lieu de trois sections d'un tiers de spire, et/ou avec des longueurs respectives L qui ne sont pas nécessairement identiques d'une portion à l'autre.

Les Figures 10 et 11 illustrent des lois de variation possibles du diamètre d sur l'étendue de la longueur L d'une des portions de fil 60a, 60b ou 60c. Cette variation est monotone et décroissante de l'extrémité distale à l'extrémité proximale de la portion, et suit une loi qui peut être linéaire (Figure 10) ou non (Figure 11); dans tous les cas il s'agit d'assurer un raccordement sans discontinuité de diamètre avec la portion suivante.

On notera que, du fait de la variation progressive du diamètre d, le fil d'hélice de la vis 26 présente sur la longueur de la région terminale 60 un pas d'enroulement à vide p variable, décroissant dans le sens distal vers proximal.

Dans un autre mode de réalisation, la raideur variable entre la partie terminale de l'hélice et la partie proximale est obtenue avec un fil de diamètre constant, mais avec un traitement différencié selon les régions du fil, par exemple un recuit thermique différencié susceptible de modifier localement la raideur du matériau constituant le fil.

Dans ce cas, le pas d'enroulement à vide p est constant, mais on peut toutefois, au moins sur la longueur de la région terminale 60, le rendre variable, décroissant dans le sens distal vers proximal.

On va maintenant décrire en référence aux Figures 12 à 14 la manière de réaliser une hélice dont la raideur en flexion est localement modifiée en donnant au fil un diamètre variable, comme décrit à la Figure 9.

Il s'agit d'obtenir, comme illustré Figure 12 (où, pour la clarté de la figure, l'échelle en direction radiale a été fortement agrandie par rapport à la direction axiale), une partie terminale 60 de diamètre variable avec côté distal, une extrémité biseautée 38 et, côté proximal, le reste du fil d'hélice 62 laissé à son diamètre d initial.

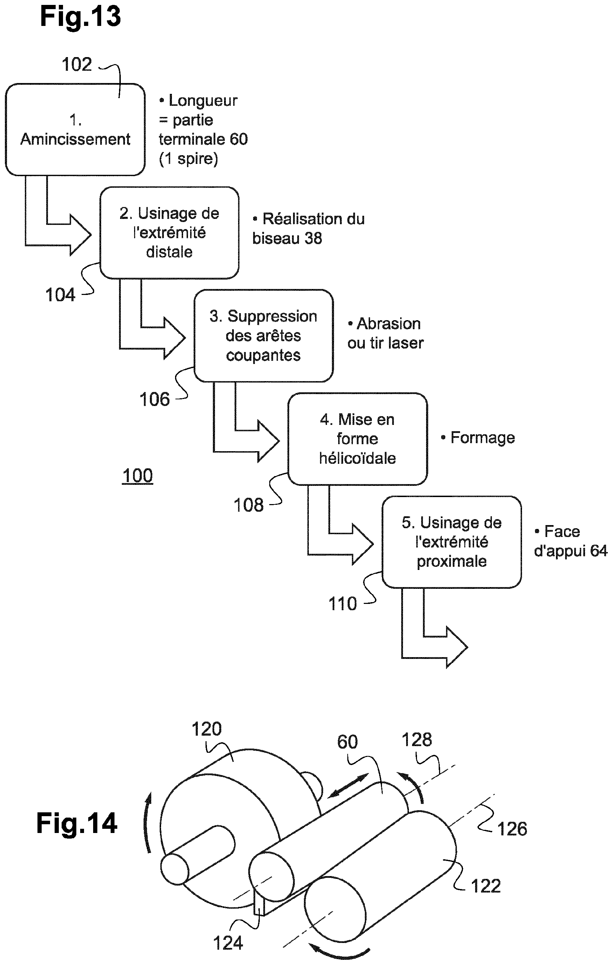

La succession des étapes est illustrée sur le schéma de processus 100 de la Figure 13.

Initialement, avant sa mise en forme hélicoïdale, le fil est un fil rectiligne. La première étape 102 consiste à opérer un amincissement progressif du diamètre de ce fil sur la longueur de la partie terminale à moindre raideur, par exemple sur une longueur de fil qui correspondra à une spire d'hélice après formage hélicoïdal.

Cet amincissement local du fil en partie distale peut être réalisé de diverses manières.

Une première technique consiste à effectuer un usinage traditionnel par un système multiaxe à translation, rotation et angulation. Le fil rectiligne est placé dans un mandrin puis mis en rotation, et l'amincissement est réalisé par usinage après orientation du porte-outil et positionnement du porte-mandrin. Pour obtenir un profil tel que celui illustré Figure 12, l'extrémité du fil est amincie suivant une forme conique, correspondant à la région terminale 60.

Une autre possibilité consiste, comme illustré Figure 14, à effectuer une rectification entre deux rouleaux abrasifs 120, 122 tournant en sens opposés. Le fil à rectifier 60 est positionné sur un support 124 qui permet d'ajuster le diamètre final. Pour obtenir la forme conique voulue sur le diamètre extérieur du fil 60, on incline l'axe de rotation 126 de l'un des cylindres abrasifs 122 par rapport à l'axe de rotation 128 du fil 60.

L'étape suivante consiste à réaliser le biseau d'extrémité 38 par enlèvement de matière (étape 104), puis supprimer les arêtes coupantes et former un arrondi par abrasion ou tir laser (étape 106).

Le fil ainsi préparé est mis en forme hélicoïdale (étape 108), par enroulement sur une machine conventionnelle de réalisation de ressorts.

Le processus se termine par une étape 110 d'usinage de l'extrémité proximale de la vis hélicoïdale pour former la face d'appui 64 (Figure 9) qui viendra en contact avec l'extrémité distale 24 du corps tubulaire 22 de la capsule implantable (Figure 3).

On va maintenant expliquer, en référence aux Figures 15 et 16, la manière dont est opérée l'implantation de la capsule au moyen de la vis selon l'invention que l'on vient de décrire.

Avec cette vis, à la différence des solutions de l'art antérieur, l'extrémité de la vis hélicoïdale est conçue de façon à ne pas perforer la paroi cardiaque.

Il s'agit essentiellement, sous l'effet du double mouvement de rotation 46 et de compression axiale 48 imprimé par l'opérateur, de faire pénétrer l'hélice dans le myocarde sans couper celui-ci (qui est très peu résistant), puis faire en sorte que l'extrémité de l'hélice vienne glisser sur le péricarde viscéral PCV sans le transpercer, créant ainsi un mode de fixation non perforant. La surface oblique inclinée 40 viendra seulement repousser le péricarde viscéral en glissant dessus, sans le percer.

La Figure 16 montre la répartition des efforts et des contraintes à l'extrémité de l'hélice.

Dans une phase initiale, lorsque l'hélice a pénétré dans le myocarde et n'a pas encore atteint le péricarde viscéral, la force d'appui axial sur le tissu cardiaque est inférieure à la raideur en compression axiale k du fil d'hélice. La force d'appui A de la surface oblique 40, combinée à la poussée radiale P produite par la rotation de la vis, produit à l'extrémité 38 du biseau une résultante R insuffisante pour traverser le péricarde viscéral PCV et risquer de l'endommager.

Une fois que la vis a atteint le péricarde viscéral PCV, la force d'appui devient supérieure à la raideur k du fil d'hélice, du fait que la résistance du péricarde viscéral PCV est plus élevée que celle du myocarde MYO. L'hélice de la vis va alors se comprimer, en écrasant le myocarde sans perforer le péricarde viscéral.

De plus, grâce au fil d'hélice à raideur en flexion variable décrit plus haut en référence aux Figures 9 à 14, une fois atteint le péricarde viscéral la partie terminale de la vis se déformera dans une géométrie non hélicoïdale du fait de la plus grande souplesse (moindre raideur à la flexion) de son extrémité. Le risque de perforation du péricarde viscéral est ainsi fortement réduit, tout en conservant pour la vis de bonnes propriétés de pénétration au moment du vissage dans le myocarde. Avantageusement, l'implant est muni d'un limiteur de couple, réalisé par exemple selon les enseignements du WO 2002/064172 A1 (US 11 197 997 B2) précité, qui permettra de débrayer l'organe d'ancrage d'avec le corps de l'implant lorsque le couple de réaction exercé par la vis dans la paroi de l'organe dépasse un couple seuil prédéterminé, évitant ainsi toute lacération des tissus.

La configuration particulière de vis hélicoïdale que l'on vient de décrire permet de disposer d'une hélice d'ancrage "intelligente", adaptative : dans le tissu fragile mais plus résistant du péricarde, le risque de perforation est fortement réduit, en gardant dans le tissu plus mou du myocarde une capacité de glissement. La longueur fixante de l'hélice est ainsi maximisée sur la quasi-totalité de l'épaisseur de la paroi de l'oreillette, bien que celle-ci soit plus mince et plus fragile que dans le cas d'une implantation ventriculaire.

## Revendications

1. Un dispositif médical implantable (10) apte à être implanté dans une paroi cardiaque auriculaire (12) comprenant successivement un endocarde (EC), un myocarde (MYO) et un péricarde viscéral (PCV), le dispositif, comprenant :
- un corps de dispositif (22) avec une face frontale (32) à son extrémité distale ; et
- un moyen d'ancrage du dispositif médical à ladite paroi cardiaque (EC, MYO, PCV),
le moyen d'ancrage comportant une vis (26) avec un fil d'hélice enroulé en une pluralité de spires non jointives (36), la vis (26) ayant une extrémité encastrée (34) solidarisée à la face frontale (32) du corps de dispositif (22) et une extrémité libre (36) comprenant une extrémité biseautée (38) définie par au moins une surface oblique (40),
***caractérisé en ce que*** le fil d'hélice de la vis (26) est un fil déformable en flexion,
*et **en ce que*** la vis (26) comprend, à son extrémité distale libre, une région terminale (60) dont la raideur en flexion du fil d'hélice est plus faible que dans une région proximale (62) des spires (36) de la vis (26),
de sorte que, lors du vissage dans une paroi cardiaque auriculaire (12), une fois atteint le péricarde viscéral (PCV), la région terminale (60) à l'extrémité distale libre de la vis (26) se déforme dans une géométrie non hélicoïdale, sans perforation du péricarde viscéral (PCV).

2. Le dispositif de la revendication 1, dans lequel la vis (26) est en outre élastiquement déformable en compression axiale,
de sorte que la vis, une fois atteint le péricarde viscéral (PCV), produise un écrasement du myocarde (MYO) sans perforation du péricarde viscéral (PCV).

3. Le dispositif de la revendication 1, dans lequel la région terminale (60) s'étend sur au moins une demi-spire de l'hélice.

4. Le dispositif de la revendication 1, dans lequel la région terminale (60) est une région de raideur non constante, comprenant une pluralité de portions successives (60a, 60b, 60c) différentes dont les raideurs respectives vont en décroissant dans un sens proximal vers distal.

5. Le dispositif de la revendication 1, dans lequel la région terminale (60) est une région de raideur non constante, comprenant un fil d'hélice de diamètre (d) variable, continûment décroissant dans un sens proximal vers distal.

6. Le dispositif de la revendication 5, dans lequel le diamètre (d) du fil d'hélice est variable sur une longueur d'une spire du fil d'hélice, puis constant sur les spires proximales suivantes de l'hélice.

7. Le dispositif de la revendication 5, dans lequel le diamètre (d) du fil d'hélice augmente de 0,2 mm côté distal de l'extrémité terminale (60), jusqu'à 0,5 mm côté proximal de l'extrémité terminale (60).

8. Le dispositif de la revendication 1, dans lequel la région terminale (60) est une région de raideur non constante, comprenant une pluralité de portions successives différentes de même diamètre ayant subi des traitements thermiques propres à modifier leurs raideurs respectives.

9. Le dispositif de la revendication 1, dans lequel le fil d'hélice de la vis (26) présente un pas d'enroulement à vide (p) constant.

10. Le dispositif de la revendication 1, dans lequel le fil d'hélice de la vis (26) présente, au moins sur la longueur de la région terminale (60), un pas d'enroulement à vide (p) variable, décroissant dans un sens distal vers proximal.

11. Le dispositif de la revendication 1, comprenant en outre un couplage débrayable agencé pour, lorsqu'une sollicitation externe en rotation axiale est appliquée au corps de dispositif (22) dans un sens de vissage de la vis (26), autoriser une rotation relative axiale entre le corps de dispositif (22) et le moyen d'ancrage dès qu'un couple de réaction exercé par la vis (26) dans la paroi (EC) de l'organe (12) du patient dépasse un couple seuil prédéterminé.

12. Le dispositif de la revendication 1, dans lequel :
- le dispositif médical est un implant cardiaque autonome de type capsule *leadless,*
- le corps de dispositif (22) loge un ensemble électronique et un module de récupération d'énergie avec un moyen de stockage d'énergie pour l'alimentation de l'ensemble électronique, et
- la face frontale (32) du corps de dispositif (22) porte une électrode (30) de détection/stimulation cardiaque apte à venir en contact avec la paroi (EC) de l'organe (12) du patient après ancrage du dispositif.

## Patentansprüche

1. Implantierbare medizinische Vorrichtung (10), die eingerichtet ist, in einer Vorhofwand (12) des Herzens implantiert zu werden, die nacheinander ein Epikard (EC), ein Myokard (MYO) und ein viszerales Perikard (PCV) umfasst, wobei die Vorrichtung umfasst:
- einen Vorrichtungskörper (22) mit einer Stirnfläche (32) an seinem distalen Ende; und
- ein Mittel zur Verankerung der medizinischen Vorrichtung an der Herzwand (EC, MYO, PCV),
wobei das Verankerungsmittel eine Schraube (26) mit einem Spiraldraht aufweist, der in einer Mehrzahl von nicht aneinander liegenden Windungen (36) gewickelt ist, wobei die Schraube (26) ein eingestecktes Ende (34) aufweist, das mit der Stirnfläche (32) des Vorrichtungskörpers (22) fest verbunden ist, und ein freies Ende (36) aufweist, das ein abgefastes Ende (38) umfasst, das von wenigstens einer schrägen Oberfläche (40) definiert ist,
***dadurch gekennzeichnet, dass*** der Spiraldraht der Schraube (26) ein in Biegung verformbarer Draht ist *und dass* die Schraube (26), an ihrem freien distalen Ende, eine Endregion (60) umfasst, deren Biegesteifigkeit des Spiraldrahts geringer ist als in einer proximalen Region (62) der Windungen (36) der Schraube (26), so dass, bei der Verschraubung in einer Vorhofwand (12) des Herzens, sobald das viszerale Perikard (PCV) erreicht wird, die Endregion (60) an dem freien distalen Ende der Schraube (26) sich in eine nicht spiralförmige Geometrie verformt, ohne Durchbohrung des viszeralen Perikard (PCV).

2. Vorrichtung nach Anspruch 1, wobei die Schraube (26) ferner in axialer Stauchung elastisch verformbar ist, so dass die Schraube, sobald sie das viszerale Perikard (PCV) erreicht, eine Stauchverformung des Myokard (MYO) ohne Durchbohrung des viszeralen Perikard (PCV) bewirkt.

3. Vorrichtung nach Anspruch 1, wobei die Endregion (60) sich über wenigstens eine halbe Windung der Spirale erstreckt.

4. Vorrichtung nach Anspruch 1, wobei die Endregion (60) eine Region mit nicht konstanter Steifigkeit ist, die eine Mehrzahl von verschiedenen aufeinanderfolgenden Abschnitten (60a, 60b, 60c) umfasst, deren jeweilige Steifigkeiten in einer proximalen Richtung zur distalen hin abnehmen.

5. Vorrichtung nach Anspruch 1, wobei die Endregion (60) eine Region mit nicht konstanter Steifigkeit ist, die einen Spiraldraht mit variablem Durchmesser (d) umfasst, der in einer proximalen Richtung zur distalen hin kontinuierlich abnimmt.

6. Vorrichtung nach Anspruch 5, wobei der Durchmesser (d) des Spiraldrahts über eine Länge einer Windung des Spiraldrahts variabel und anschließend über die folgenden proximalen Windungen der Spirale konstant ist.

7. Vorrichtung nach Anspruch 5, wobei der Durchmesser (d) des Spiraldrahts von 0,2 mm auf der distalen Seite des Endes (60) bis auf 0,5 mm auf der proximalen Seite des Endes (60) zunimmt.

8. Vorrichtung nach Anspruch 1, wobei die Endregion (60) eine Region mit nicht konstanter Steifigkeit ist, die eine Mehrzahl von verschiedenen aufeinanderfolgenden Abschnitten mit gleichem Durchmesser umfasst, die thermischen Behandlungen zur Veränderung ihrer jeweiligen Steifigkeiten unterzogen wurden.

9. Vorrichtung nach Anspruch 1, wobei der Spiraldraht der Schraube (26) unbelastet eine konstante Windungshöhe (*p*) aufweist.

10. Vorrichtung nach Anspruch 1, wobei der Spiraldraht der Schraube (26), wenigstens über die Länge der Endregion (60), unbelastet eine variable Windungshöhe (*p*) aufweist, die in einer distalen Richtung zur proximalen hin abnimmt.

11. Vorrichtung nach Anspruch 1, ferner umfassend eine auskuppelbare Kupplung, die angeordnet ist, um, wenn eine äußere axiale Rotationsbelastung auf den Vorrichtungskörper (22) in einer Verschraubungsrichtung der Schraube (26) aufgebracht wird, eine axiale relative Rotation zwischen dem Vorrichtungskörper (22) und dem Verankerungsmittel zu gestatten, sobald ein Reaktionsmoment, das von der Schraube (26) in der Wand (EC) des Organs (12) des Patienten ausgeübt wird, einen vorbestimmten Schwellwert übersteigt.

12. Vorrichtung nach Anspruch 1, wobei:
- die medizinische Vorrichtung ein autonomes Herzimplantat vom Typ *leadless-Kapsel* ist,
- der Vorrichtungskörper (22) eine elektronische Anordnung und ein Energierückgewinnungsmodul mit einem Energiespeichermittel für die Versorgung der elektronischen Anordnung aufnimmt und
- die Stirnseite (32) des Vorrichtungskörpers (22) eine Elektrode (30) zur Erfassung/Stimulation des Herzschlags trägt, die dazu eingerichtet ist, nach Verankerung der Vorrichtung mit der Wand (EC) des Organs (12) des Patienten in Kontakt zu kommen.

## Claims

1. An implantable medical device (10) intended to be implanted into a atrial heart wall (12) comprising successively an endocardium (EC), a myocardium (MYO) and a visceral pericardium (PCV), the device comprising:
- a device body (22) with a front face (32) at its distal end; and
- a means for anchoring the medical device to said heart wall (EC, MYO, PCV),
wherein the means for anchoring includes a screw (26) with a helix wire wound into a plurality of non-contiguous turns (36), the screw having a clamped end (34) integral with the front face (32) of the device body (22) and a free end (36) comprising a beveled end (38) defined by at least one oblique surface (40),
***characterised in that*** the helix wire of the screw (26) is a wire that is deformable in bending,
*and **in that*** the screw (26) comprises, at its free distal end, a terminal region (60) whose helix wire bending stiffness is lower than in a proximal region (62) of the turns (36) of the screw (26),
whereby, during a screwing into an atrial heart wall (12), once the visceral pericardium (PCV) reached, the terminal region (60) at the free distal end of the screw (26) is deformed according to a non-helical geometry, without piercing of the visceral pericardium (PCV).

2. The device of claim 1, wherein the screw (26) is further elastically deformable in axial compression,
whereby the screw, once the visceral pericardium (PCV) reached, produces a crushing of the myocardium (MYO) without piercing of the visceral pericardium (PCV).

3. The device of claim 1, wherein the terminal region (60) extends over at least one half-turn of the helix.

4. The device of claim 1, wherein the terminal region (60) is a region of non-constant stiffness, comprising a plurality of different successive portions (60a, 60b, 60c) whose respective stiffnesses decrease in a proximal to distal direction.

5. The device of claim 1, wherein the terminal region (60) is a region of non-constant stiffness, comprising a helix wire of a variable diameter (d) continuously decreasing in a proximal to distal direction.

6. The device of claim 5, wherein the diameter (d) of the helix wire is variable over a length of one helix wire turn, then constant over the following proximal turns of the helix.

7. The device of claim 5, wherein the diameter (d) of the helix wire increases by 0.2 mm on the distal side of the terminal end (60), up to 0.5 mm on the proximal side of the terminal end (60).

8. The device of claim 1, wherein the terminal region (60) is a region of non-constant stiffness, comprising a plurality of different successive portions of same diameter having undergone heat treatments for modifying their respective stiffnesses.

9. The device of claim 1, wherein the helix wire of the screw (26) has a constant winding pitch (p) under zero-load condition.

10. The device of claim 1, wherein the helix wire of the screw has, at least over the length of the terminal region, a variable no-load winding pitch, decreasing in a distal to proximal direction.

11. The device of claim 1, further comprises a disengageable coupling arranged in such a way as, when an external axial rotational stress is applied to the device body (22) in a screwing direction of the screw (26), to allow a relative axial rotation between the device body (22) and the means for anchoring as soon as a reaction torque exerted by the screw (26) in the wall (EC) of the patient's organ (12) exceeds a predetermined threshold torque.

12. The device of claim 1, wherein:
- the medical device is an autonomous cardiac implant of the leadless capsule type,
- the device body (22) houses an electronic unit and an energy harvesting module with an energy storage means for powering the electronic unit, and
- the front face (32) of the device body (22) carries a cardiac sensing/pacing electrode (30) adapted to come into contact with the wall (EC) of the patient's organ (12) after anchoring of the device.
